# EUROPEAN PATENT APPLICATION

(11) **EP 2 163 270 A1**
(43) Date of publication of application: **17.03.2010**
(21) Application number: 08764097.5
(22) Date of filing: 12.06.2008
(51) Int. Cl.: A61M 1/14, A61M 1/34

(54) **DOUBLE FILTRATION BLOOD PURIFICATION APPARATUS AND METHOD OF PRIMING THEREFOR**

(30) Priority: 13.06.2007 JP 2007156638
(71) Applicant: Kuraray Medical Inc., Kurashiki-shi Okayama 7100801 (JP)
(72) Inventor: INOUE, Masao, Okayama 7048191 (JP); IKE, Akihiro, Tokyo 100-0004 (JP)
(74) Representative: Jenkins, Peter David
(86) International application number: PCT/JP2008/001502
(87) International publication number: WO 2008/152809

(57) **Abstract**

To provide a double filtration blood purification apparatus that can be primed while generation of bubbles within each of a separating membrane in both of a blood component separator and a plasma component separator is avoided and also to provide a method of priming such blood purification apparatus. A cleansing liquid introducing passage 19 is provided, which is a passage dedicated to introduce a cleansing liquid P from a cleansing liquid supply source 57 into a plasma component separator 3 and, also, this cleansing liquid introducing passage 19 is provided with a dedicated, third pump 3. When the third and first pumps are driven in normal and reverse directions, respectively, by a controller 50, a sufficient pressure is applied to the cleansing liquid P by the third ump and the cleansing liquid P can be introduced into the plasma component separator 3.

## Description

### CROSS REFERENCE TO THE RELATED APPLICATION

This application is based on and claims priority to Japanese patent application No. 2007-156638, filed June 13, 2007, the entire disclosure of which is herein incorporated by reference as a part of this application.

### BACKGROUND OF THE INVENTION

### (Field of the Invention)

The present invention relates to a double filtration blood purification apparatus including a blood component separator for separating blood plasma from blood and a plasma component separator for separating a high molecular weight subcomponent from the separated blood plasma, and a method of priming such blood purification apparatus.

### (Description of the Related Art)

As an apparatus used in medical treatment of patients suffering from, for example, liver failure or autoimmune disorder, the double filtration blood purification apparatus has been well known, which is operable to separate the blood into blood cells and a blood plasma component by means of a blood component separator and then separating the separated blood plasma component into a low molecular weight subcomponent and a high molecular weight subcomponent containing toxins by means of a plasma component separator. In this kind of apparatus, a so-called priming operation is required prior to the medical treatment, in which a fluid passage through which the blood flows, the blood component separator and the plasma component separator are rinsed and filled with a cleaning liquid such as, for example, physiologic saline.

As such a priming process, the process has been well known, which includes driving a blood pump in a negative direction, reverse to a positive direction, to introduce the cleansing liquid into the blood component separator to purge air inside the blood component separator to the outside of the apparatus, driving the blood pump in the positive direction together with a drain pump (a plasma discharge pump) to introduce the cleansing liquid into the plasma component separator to purge air inside the plasma component separator to the outside of the apparatus, to thereby rinse and refill the blood component separator, the plasma component separator and the various fluid passages with the cleansing liquid. For the details thereof, see the Patent Document 1 listed below.

Another priming process has also been well known, which includes pressurizing a filling liquid within the blood component separator to urge the cleansing liquid to a junction between a blood return passage and a plasma return passage, causing a cleansing liquid to flow from a cleansing liquid supply source to the junction past a return blood drip chamber disposed in the blood return passage to that the refilling liquid and the cleansing liquid can be merged with each other, driving a blood pump, a plasma separating pump and a liquid discharge pump in a positive direction or a negative direction to allow the cleansing liquid to flow through the blood component separator, the plasma component separator and a plasma separating passage to complete the cleansing and refilling. For the detail thereof, see the Patent Document 2 listed below.
[Patent Document 1] JP Patent No. 1533117
[Patent Document 2] JP Laid-open Patent Publication No. 2005-253555

### SUMMARY OF THE INVENTION

It has, however, been found that the priming process disclosed in the Patent Document 1 listed above has the following problem. Specifically, when the cleansing liquid is supplied across the plasma component separator, it may often occur that air inside the fluid passage may mix into a separation membrane of the plasma component separator, but the drain pump used to discharge the cleansing liquid, introduced into the plasma component separator through a plasma flow passage, to the outside of the apparatus concurrently assume the role of introducing the cleansing liquid from the cleansing liquid supply source directly into the outside of the separation membrane of the plasma component separator and, therefore, the drain pump can be sufficiently pressurized enough to purge the air admixed in the separation membrane. As a result thereof, the surface area of the separation membrane tends to be reduced in the presence of the air and the separating performance of the plasma component separator is accordingly lowered.

On the other hand, the priming process disclosed in the Patent Document 2 listed above has such a problem that since the plasma separating pump is reversed to rotate in the negative direction to draw the cleansing liquid to flow into the plasma separating passage from the plasma return passage through the plasma component separator, the total distance of the path of flow of the cleansing liquid tends to be large enough to lower the pressure inside each of the plasma component separator, the plasma return passage and the plasma separating passage, that is, enough to make it easy to develop a negative pressure. As a result thereof, the air inside the separation membrane of the plasma component separator tends to be eluted and/or an external air tends to admix from the junction of the fluid passages, accompanied by development of bubbles inside the separation membrane of the plasma component separator and the various fluid passages and/or damages to the separation membrane. Therefore, there is the risk that the separating performance of the plasma component separator may be lowered and/or the performance of the blood purification apparatus in its entirety may be lowered.

In view of the foregoing, the present invention has been devised to substantially eliminate the above discussed problems and inconveniences inherent in the prior art blood purification apparatuses and is intended to provide a double filtration blood purification apparatus that can be primed with the air purged away from the plasma component separator and the fluid passages while generation of bubbles within a separation membrane of the blood component separator and the separation membrane of the plasma component separator is prevented.

Another important object of the present invention is to provide a method of priming the blood purification apparatus of the kind referred to above.

In order to accomplish those objects of the present invention, there is provided a double filtration blood purification apparatus, which includes a blood component separator for separating a blood into a blood cell component and a plasma component, a plasma component separator for separating the plasma component into a high molecular weight subcomponent and a low molecular weight subcomponent, a plasma introducing passage fluidly connected with the blood component separator and the plasma component separator for introducing the plasma component into the plasma component separator, a high molecular plasma passage for discharging the high molecular weight subcomponent, which has been so separated, from the plasma component separator, a cleansing liquid introducing passage fluidly connected with the plasma component separator for introducing a cleansing liquid into the plasma component separator, a first pump disposed in the plasma introducing passage, a second pump disposed in the high molecular plasma passage, a third pump disposed in the cleansing liquid introducing passage, and a controller for controlling the first, second and third pumps, in which the controller referred to above is so operable during a priming as to drive the third pump and the first pump in a normal direction and a reverse direction, respectively, to allow the cleansing liquid to flow from the cleansing liquid introducing passage into the plasma component separator and then from the plasma component separator to the blood component separator through the plasma introducing passage.

According to another aspect of the present invention, there is provided a method of priming the double filtration blood purification apparatus of the type referred to above, which method includes separating a blood into a blood cell component and a plasma component by means of a blood component separator, introducing the plasma component, which has been so separated, into a plasma component separator through a plasma introducing passage, separating the plasma component into a high molecular weight subcomponent and a low molecular weight subcomponent by means of the plasma component separator, discharging the high molecular weight subcomponent, which has been so separated, from the plasma component separator through the high molecular plasma passage, introducing a cleansing liquid from a cleansing liquid introducing passage into the plasma component separator, providing a first pump in the plasma introducing passage, providing a second pump in the high molecular plasma passage, providing a third pump in the cleansing liquid introducing passage, and, during the priming, driving the third pump and the first pump in a positive direction and a negative direction, respectively, to allow the cleansing liquid to flow from the cleansing liquid introducing passage into the plasma component separator and then from the plasma component separator into the blood component separator through the plasma introducing passage.

According to the apparatus of the present invention and the method of the present invention, the cleansing liquid introducing passage is provided, which is a passage dedicated to introduce the cleansing liquid from the cleansing liquid supply source into the plasma component separator, and this cleansing liquid introducing passage is provided with a dedicated, third pump for transporting only the cleansing liquid during the priming procedure and, therefore, when the third pump and the first pump are driven in the normal and reverse directions, respectively, by the controller, a sufficient pressure can be applied to the cleansing liquid and the cleansing liquid can be introduced into the plasma component separator and the blood component separator. In view of this, air inside the plasma component separator can be urged away. Also, since development of a negative pressure inside the plasma component separator and the plasma introducing passage can be avoided, elution of air from inside of the system including the separating membrane and intrusion of an external air from junctions of the passages can be avoided. Accordingly, the plasma component separator and the blood component separator can fully exhibit their separating capabilities and, hence, the double filtration blood purification apparatus can exhibit a sufficient purifying capability.

In a preferred embodiment of the present invention, the double filtration blood purification apparatus and the priming method are provided with an air valve for discharging air from the blood component separator to the outside of the apparatus, which air valve is closed by the controller during the priming procedure. By this construction, the air purged outwardly from the plasma component separator can, after the pressure thereof has been increased by the first pump then driven in the reverse direction, introduced into the blood component separator through the first pump and is then discharged smoothly from the air valve to the outside of the apparatus.

According to another preferred embodiment of the present invention, the delivery of the third pump may be set to be larger than that of the first pump. By so setting, a sufficient pressure can be applied to the cleansing liquid so that the latter can be introduced from the plasma component separator into the blood component separator through the plasma introducing passage and, in other words, the first pump does not draw the cleansing liquid within the plasma component separator into the plasma introducing passage in excess of the delivery of the third pump. Therefore, development of a negative pressure inside the plasma component separator or the plasma introducing passage can be avoided. As a result thereof, development of bubbles within the plasma introducing passage and the plasma component separator can be avoided, wherefore reduction in performance of the plasma component separator, which would occur when the bubbles remain within the plasma component separator or the blood component separator, can be avoided.

According to a further preferred embodiment of the present invention, the cleansing liquid introducing passage may be so set as to be selectively connected with a liquid replacement supply source for supplying a liquid replacement to the low molecular weight subcomponent during a clinical treatment. This is particularly advantageous in that the additional use of a dedicated passage for adding the liquid replacement to the low molecular weight subcomponent can be dispensed with and, therefore, the double filtration blood purification apparatus can be simplified in structure with improvement in clinical performance.

In a yet preferred embodiment of the present invention, the separated plasma component may be introduced from upper side into the plasma component separator. This is particularly advantageous in that since the plasma component can be introduced from upper side into the plasma component separator so as to flow upwardly through the plasma component separator, the efficiency of separation of the plasma component into the high molecular weight subcomponent and the low molecular weight subcomponent can be increased.

### BRIEF DESCRIPTION OF THE DRAWINGS

In any event, the present invention will become more clearly understood from the following description of preferred embodiments thereof, when taken in conjunction with the accompanying drawings. However, the embodiments and the drawings are given only for the purpose of illustration and explanation, and are not to be taken as limiting the scope of the present invention in any way whatsoever, which scope is to be determined by the appended claims. In the accompanying drawings, like reference numerals are used to denote like parts throughout the several views, and:
Fig. 1 is a schematic diagram showing a heated double filtration blood purification apparatus in an operative condition assumed during a blood treatment performed thereby;
Fig. 2 is a schematic diagram showing the heated double filtration blood purification apparatus, shown in Fig. 1, in an operative condition assumed a priming operation;
Fig. 3 is a fluid passage diagram showing the manner in which the heated double filtration blood purification apparatus of Fig. 3 is primed;
Fig. 4 is a fluid passage diagram showing the manner in which the heating type double filtration blood purification apparatus of Fig. 3 is primed;
Fig. 5 is a fluid passage diagram showing the manner in which the heating type double filtration blood purification apparatus of Fig. 3 is primed;
Fig. 6 is a fluid passage diagram showing the manner in which the heating type double filtration blood purification apparatus of Fig. 3 is primed;
Fig. 7 is a fluid passage diagram showing the manner in which the heating type double filtration blood purification apparatus of Fig. 3 is primed;
Fig. 8 is a fluid passage diagram showing the manner in which the heating type double filtration blood purification apparatus of Fig. 3 is primed;
Fig. 9 is a fluid passage diagram showing the manner in which the heating type double filtration blood purification apparatus of Fig. 3 is primed;
Fig. 10 is a fluid passage diagram showing the manner in which the heating type double filtration blood purification apparatus of Fig. 3 is primed;
Fig. 11 is a fluid passage diagram showing the manner in which the heating type double filtration blood purification apparatus of Fig. 3 is primed;
Fig. 12 is a schematic diagram showing the double filtration blood purification apparatus according to another preferred embodiment of the present invention in the operative condition assumed the blood treatment performed thereby;
Fig. 13 is a schematic diagram showing the double filtration blood purification apparatus, shown in Fig. 12, in the operative condition assumed the priming operation;
Fig. 14 is a schematic diagram showing the manner of priming the double filtration blood purification apparatus shown in Fig. 13;
Fig. 15 is a schematic diagram showing the manner of priming the double filtration blood purification apparatus shown in Fig. 13;
Fig. 16 is a schematic diagram showing the manner of priming the double filtration blood purification apparatus shown in Fig. 13;
Fig. 17 is a schematic diagram showing the manner of priming the double filtration blood purification apparatus shown in Fig. 13;
Fig. 18 is a schematic diagram showing the manner of priming the double filtration blood purification apparatus shown in Fig. 13;
Fig. 19 is a schematic diagram showing the manner of priming the double filtration blood purification apparatus shown in Fig. 13;
Fig. 20 is a schematic diagram showing the manner of priming the double filtration blood purification apparatus shown in Fig. 13; and
Fig. 21 is a schematic diagram showing the manner of priming the double filtration blood purification apparatus shown in Fig. 13.

### DETAILED DESCRIPTION OF THE EMBODIMENTS

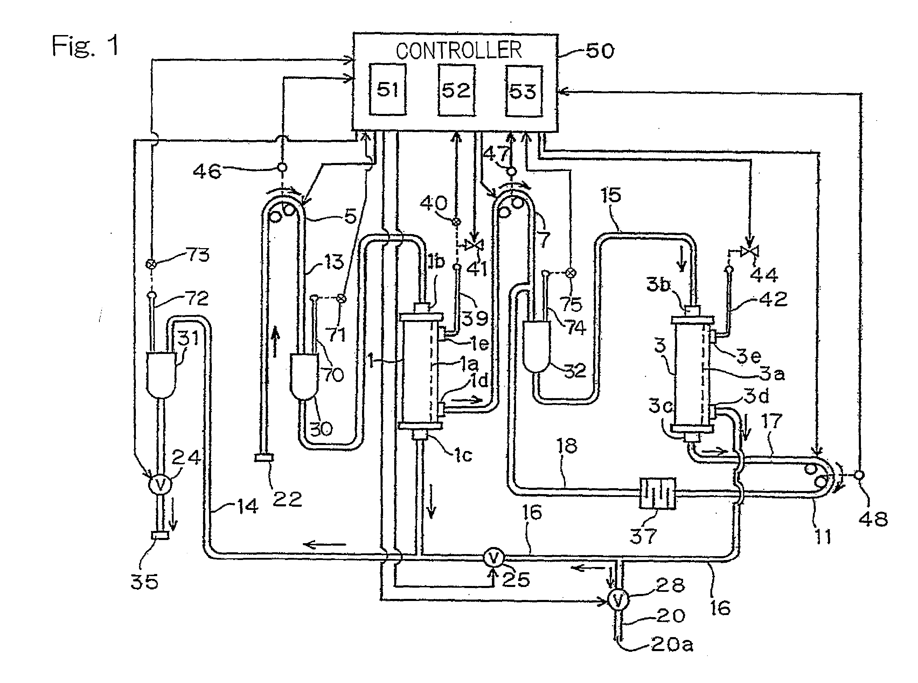

Hereinafter, the present invention will be described in detail in connection with a preferred embodiment thereof with reference to the accompanying drawings. In the first place, a double filtration blood purification apparatus, by which a priming method of the present invention is executed, will be described. Fig. 1 illustrates a schematic diagram showing a heating type double filtration blood purification apparatus in an operative condition assumed during a blood treatment performed thereby. This double filtration blood purification apparatus is of a double filtration type and includes a blood component separator 1 for separating the blood into a blood cell component and a plasma component, and a plasma component separator 3 for separating the plasma component, which has been so separated by the blood component separator 1, into a low molecular weight subcomponent and a high molecular weight subcomponent containing toxins.

Each of the blood component separator 1 and the plasma component separator 3 is in the form of, for example, a cylindrical housing having a hollow fiber flat plate, or tubular separating membrane accommodated therein. The separating membrane 1a of the blood component separator 1 has a multiplicity of pores defined therein and having a pore size within the range of 0.1 to 0.5 µm, preferably about 0.2 µm and is employed in the form of a homogeneous microporous membrane, a microfiltration membrane or an asymmetric membrane made up of a porous support layer and a microporous structural layer. Although various separating membranes have been well known as a membrane 1a for the blood component separator 1, the use is preferred of the separating membrane having an excellent biocompatibility, made of a copolymer of a polyvinyl alcohol (PVA) system, a copolymer of an ethylene vinyl alcohol (EVA) system, a cellulose derivative or polysulfone or the like. On the other hand, the separating membrane 3a of the plasma component separator 3 has a multiplicity of pores defined therein and having a pore size within the range of 0.01 to 0.04 micrometer, preferably about 0.02 µm and is employed in the form of, for example, a homogeneous microporous membrane, a microfiltration membrane or an asymmetric membrane made up of a porous support layer and a microporous structural layer. Although as is the case with the separating membrane 1a of the blood component separator 1, various separating membranes have been well known as a membrane 3a for the plasma component separator 3, the use is preferred of the separating membrane having an excellent biocompatibility, made of a copolymer of the EVA system or a cellulose derivative or the like. In the illustrated embodiment, each of the blood component separator 1 and the plasma component separator 3 is a wet type including a cylindrical housing having a multiplicity of tubular hollow fiber membranes accommodated therein and also having a filling liquid filled therein and disposed with its longitudinal axis oriented vertically.

The blood component separator 1 is fluidly connected with a blood introducing passage 13 for introducing therethrough a blood, drawn from a blood introducing element 22 (an element that can be communicated with an ordinary blood collecting vessel such as, for example, a shunt or a syringe needle, or a blood reservoir), into the blood component separator 1, and the blood introducing passage 13 is in turn fluidly connected with a blood pump 5 and then with a blood drip chamber 30, both disposed in an upstream portion of the blood introducing passage 13 with respect to the direction of flow of the blood. The blood introduced from the blood introducing element 22 into the blood introducing passage 13 is, after the pressure thereof has been increased by the blood pump 5, supplied into the blood drip chamber 30. Subsequently the blood so supplied into the blood drip chamber 30 is supplied dropwise from the blood drip chamber 30 into the blood component separator 1 by way of a blood inlet 1b, defined in an upper (upstream) end portion thereof, so that the blood can be separated by the separating membrane 1a into a blood cell component and a plasma component.

The blood component separator 1 is also fluidly connected with a blood return passage 14 through which the blood cell component separated from the blood in the manner described above may be returned to a patient's body. This blood return passage 14 is provided with, from an upstream side thereof, a return blood drip chamber 31 and then with a return blood valve 24. The blood cell component so separated emerges outwardly from the blood component separator 1 by way of a blood outlet 1c defined in a lower (downstream) end portion thereof and is then supplied into the return blood drip chamber 31. Thereafter, the blood cell component is dropwise supplied from the return blood drip chamber 31 and is, after having been introduced to a blood delivery element 35 (an element that can be communicated with a shunt or a intravenous drip kit) by way of a return blood valve 24 then opened, returned to the patient's body.

The plasma component separator 3 is fluidly connected with the blood component separator 1 through a plasma introducing passage 15, which extends from a plasma outlet 1d, defined in a side surface of the blood component separator 1 proximate to the blood outlet 1c, to a plasma inlet 3b defined in an upper (upstream) end portion of the plasma component separator 3. The plasma introducing passage 15 is provided with a plasma introducing pump 7, forming a first pump, and a plasma drip chamber 32, both disposed in an upstream portion of such plasma introducing passage 15. The plasma component emerging outwardly from the plasma outlet 1d of the blood component separator 1 is, after the pressure thereof has been increased by the plasma introducing pump 7, introduced into the plasma drip chamber 32. Subsequently, the plasma component supplied dropwise from the plasma drip chamber 32 is introduced into the plasma component separator 3 from upper side by way of a plasma inlet 3b so that the plasma component may be separated into a low molecular weight subcomponent and a high molecular weight subcomponent. Since the plasma component is introduced from upper side into the plasma component separator 3 as described above, separation of the plasma component can be efficiently accomplished by the effect of a gravitational force. Each of the drip chambers 30, 31 and 32 referred to above is capable of pooling, in an upper region thereof, air trapped from the associated fluid passage and enabling the pressure inside the associated fluid passage to be detected through the air pooled in that upper region thereof.

The blood drip chamber 30 is fluidly connected with a third air line 70 for the detection of a pressure, which line 70 is provided with a first inlet pressure sensor 71 for detecting an inlet pressure of the blood component separator 1. Similarly, the return blood drip chamber 31 is fluidly connected with a fourth air line 72 for the detection of a pressure, which line 72 is provided with a return blood pressure sensor 73 for detecting a patient return blood pressure. Again, the plasma drip chamber 32 is fluidly connected with a fifth air line 74 for the detection of a pressure, which line 74 is provided with a second inlet pressure sensor 75 for detecting an inlet pressure of the plasma component separator 3.

The plasma component separator 3 has a lower (downstream) end portion formed with a first plasma component outlet 3c for delivering the separated high molecular weight subcomponent and also has a portion of a side surface proximate to the first plasma component outlet 3c formed with a second plasma component outlet 3d for delivering the separated low molecular weight subcomponent. A high molecular plasma passage 17 for discharging the separated high molecular weight subcomponent from the plasma component separator 3 is fluidly connected with the first plasma component outlet 3c of the plasma component separator 3. The separated high molecular weight subcomponent contains a plasma component of a kind, which would not require any disposal if filtration thereof is repeated, that is, a useful plasma component of a kind that can be returned to the patient's body, and, as a passage for separating such a useful plasma component, a plasma recirculating passage 18 for recirculating the high molecular weight subcomponent back to the plasma component separator 3 is employed. The high molecular plasma passage 17 referred to above is provided with a plasma discharge pump 11, forming a second pump, and a heater 37 in the form of an electric heater, the plasma discharge pump 11 being positioned upstream of the heater 37. The high molecular plasma passage 17 has its downstream portion fluidly connected with a portion of the plasma introducing passage 15 between a plasma introducing pump 7 and a plasma drip chamber 32.

The high molecular weight subcomponent of the plasma discharged from the first plasma component outlet 3c of the plasma component separator 3 is, after the pressure thereof has been increased by the plasma discharge pump 11, supplied into the high molecular plasma passage 17 and is then recirculated to the plasma introducing passage 15 through the heater 37. Thereafter, the high molecular weight subcomponent is introduced into the plasma component separator 3 by way of the plasma drip chamber 32.

On the other hand, a plasma return passage 16 for returning the separated low molecular weight subcomponent back to the patient's body extends from the second plasma component outlet 3d and is merged with the blood return passage 14 at a location between the blood outlet 1c of the blood component separator 1 and the return blood drip chamber 31. This plasma return passage 16 is provided with a plasma return valve 25 for selectively opening or closing the fluid circuit of the plasma return passage 16. When this plasma return valve 25 is set in a position to open the fluid circuit of the plasma return passage 16, the low molecular weight subcomponent emerging outwardly from the second plasma component outlet 3d can be returned to the patient's body through the plasma return passage 16 and then through the blood return passage 14. Also, a portion of the plasma return passage 16 between the second plasma component outlet 3d of the plasma component separator 3 and the plasma return valve 25 is fluidly connected with a branch passage 20. This branch passage 20 is provided with a cleansing liquid discharge valve 28 for selectively opening or closing the communication with the outside of the apparatus and, as will be described in detail later, when this cleansing liquid discharge valve 28 is set in position to open the communication with the outside of the apparatus during the priming, the cleansing liquid can be discharged to the outside through a cleansing liquid discharge port 20a. The branch passage 20 is preferably positioned at a site lower in level than any other passages so that the cleansing liquid can be smoothly discharged to the outside of the apparatus.

A portion of a side surface of the blood component separator 1 proximate to the blood inlet 1b is formed with a pressure detecting port le, which is fluidly connected with a first air line 39. This first air line 39 is in turn provided with a first filtering pressure sensor 40 for detecting a membrane pressure of the separating membrane 1a in the blood component separator 1 and a first air valve 41 for selectively opening or closing the communication with the outside of the apparatus. Accordingly, when the first air valve 41 is set in a position to open the communication with the outside of the apparatus, air can be introduced or discharged between the blood component separator 1 and the outside of the apparatus.

Similarly, a portion of a side surface of the plasma component separator 3 proximate to the plasma inlet 3b is formed with a cleansing liquid inlet 3e, which is fluidly connected with a second air line 42. This second air line 42 is in turn provided with a second air valve 44 for selectively opening or closing the communication with the outside of the apparatus. Accordingly, when the second air valve 44 is set in a position to open the communication with the outside of the apparatus, air can be introduced or discharged between the plasma component separator 3 and the outside of the apparatus.

Also, the blood drip chamber 30 is fluidly connected with a third air line 70 for the detection of a pressure, and this third air line 70 is provided with a first inlet pressure sensor 71 for detecting an inlet pressure of the blood component separator 1. Similarly, the return blood drip chamber 31 is fluidly connected with a fourth air line 72 for the detection of a pressure, and this fourth air line 72 is provided with a return blood pressure sensor 73 for detecting a patient return blood pressure. Furthermore, the plasma drip chamber 32 is fluidly connected with a fifth air line 74, and this fifth air line 74 is provided with a second inlet pressure sensor 75 for detecting an inlet pressure of the plasma component separator 3.

In addition, the blood pump 5 in the blood introducing passage 13 is connected with a blood flow sensor 46 for detecting the flow in the blood introducing passage 13 based on the delivery (the number of revolutions) of the blood pump 5; the plasma introducing pump 7 in the plasma introducing passage 15 is connected with a plasma flow sensor 47 for detecting the flow in the plasma introducing passage 15 based on the delivery of the plasma introducing pump 7; and the plasma discharge pump 11 in the high molecular plasma passage 17 is connected with a plasma discharge sensor 48 for detecting the flow in the high molecular plasma passage 17 based on the delivery of the plasma discharge pump 11.

The heating type double filtration blood purification apparatus of the construction hereinabove described is provided with a controller 50 for controlling the sequence of purifying the blood. This controller 50 includes a blood pump drive unit 51 for driving the blood pump 5, a plasma introducing pump drive unit 52 for driving the plasma introducing pump 7 and a plasma discharge pump drive unit 53 for driving the plasma discharge pump 11, all built therein. During the treatment of the blood to purify the latter, by the operation of the controller 50, based on respective detected flow signals, which are fed from the blood flow sensor 46, the plasma flow sensor 47 and the plasma discharge sensor 48, and respective detected pressure signals, which are fed from the first filtering pressure sensor 40 in the first air line 39, the first inlet pressure sensor 71 in the third air line 70, the return blood sensor 73 in the fourth air line 72 and the second inlet pressure sensor 75 in the fifth air line 74, respective deliveries of the blood pump 5, the plasma introducing pump 7 and the plasma discharge pump 11 are controlled by the blood pump drive unit 51, the plasma introducing pump drive unit 52 and the plasma discharge pump unit 53 so that respective flows in the various passages and respective membrane pressures inside the blood component separator 1 and the plasma component separator 3 can attain proper values, to thereby complete the treatment of the blood. Although not shown, the controller 50 of the type described above has valve drive units built therein for driving the return blood valve 24, the return plasma valve 25, the cleansing liquid discharge valve 28, the first air valve 41 and the second air valve 44, respectively.

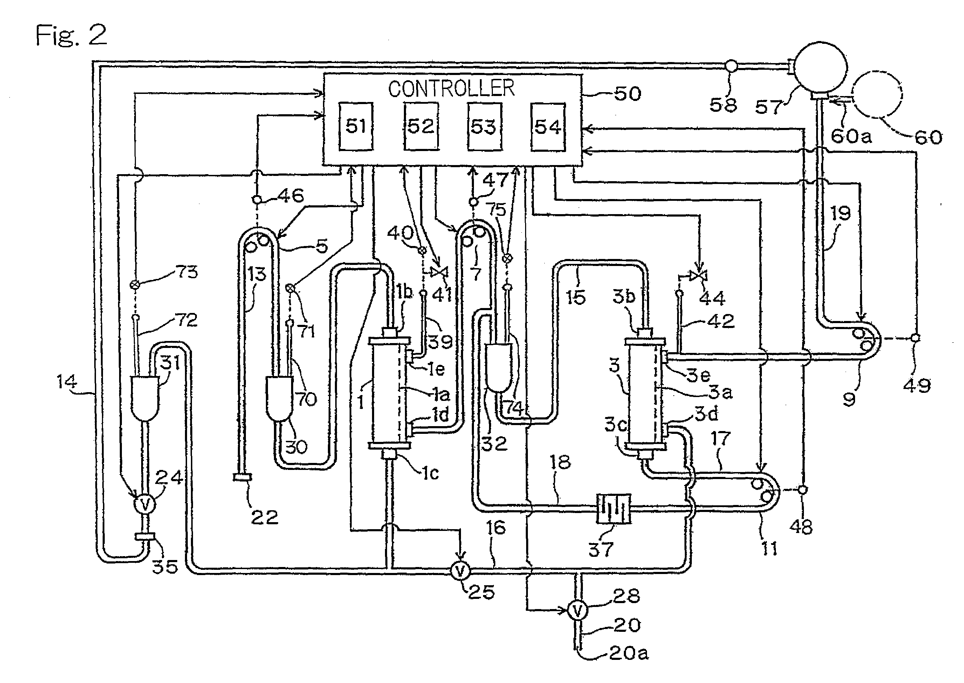

Fig. 2 illustrates a schematic diagram showing the manner of connection of the fluid passages that is employed in the heating type double filtration blood purification apparatus of Fig. 1 when the priming method of the present invention is performed. As shown therein, in order to perform the priming of the fluid circuit of the double filtration blood purification apparatus, the cleansing liquid inlet 3e of the plasma component separator 3 is fluidly connected not only with the second air line 42 as hereinbefore described, but with a cleansing liquid introducing passage 19 through which the cleansing liquid such as, for example, physiological saline is introduced from a cleansing liquid supply source 57 into the plasma component separator 3. This cleansing liquid introducing passage 19 is provided with a cleansing liquid pump 9 forming a third pump. During the priming, the cleansing liquid emerging outwardly from the cleansing liquid supply source 57 is, after the pressure thereof has been increased by the cleansing liquid pump 9, introduced into the plasma component separator 3 by way of the cleansing liquid inlet 3e. The cleansing liquid supply source 57 is preferably disposed at a site higher in level than any other fluid passages so that the cleansing liquid can be introduced by the effect of the gravitational force. Also, during the blood treatment, a blood delivery element 35 of the blood return passage 14, which is fluidly connected with, for example, a shunt or dropper kit, is fluidly connected with the cleansing liquid supply source 57 through a bubble sensor 58.

A cleansing liquid flow sensor 49 for detecting the flow in the cleansing liquid introducing passage 19 on the basis of the delivery of the cleansing liquid pump 9 is connected with the cleansing liquid pump 9 in the cleansing liquid introducing passage 19. Also, the controller 50 referred to above has a cleansing liquid pump driving unit 54 built therein for driving the cleansing liquid pump 9. During the priming, by the controller 50, based on respective detected flow signals, which are fed from the blood flow sensor 46, the plasma flow sensor 47, the plasma discharge sensor 48 and the cleansing liquid flow sensor 49, and respective detected pressure signals, which are fed from the first filtering pressure sensor 40 in the first air line 39, the first inlet pressure sensor 71 in the third air line 70, the return blood sensor 73 in the fourth air line 72, and the second inlet pressure sensor 75 in the fifth air line 74, respective deliveries of the blood pump 5, the plasma introducing pump 7, the plasma discharge pump 11 and the cleansing liquid pump 9 are controlled so that respective flows in the various passages and respective membrane pressures inside the blood component separator 1 and the plasma component separator 3 can attain proper values, to thereby complete the treatment of the blood.

It is to be noted that the heating type double filtration blood purification apparatus of the structure described above may be provided with various accessories including, for example, an inspirator of anticoagulant such as, for example, heparin for preventing coagulation of the blood, which is disposed in communication with the blood drip chamber 30, but the details thereof are not herein described.

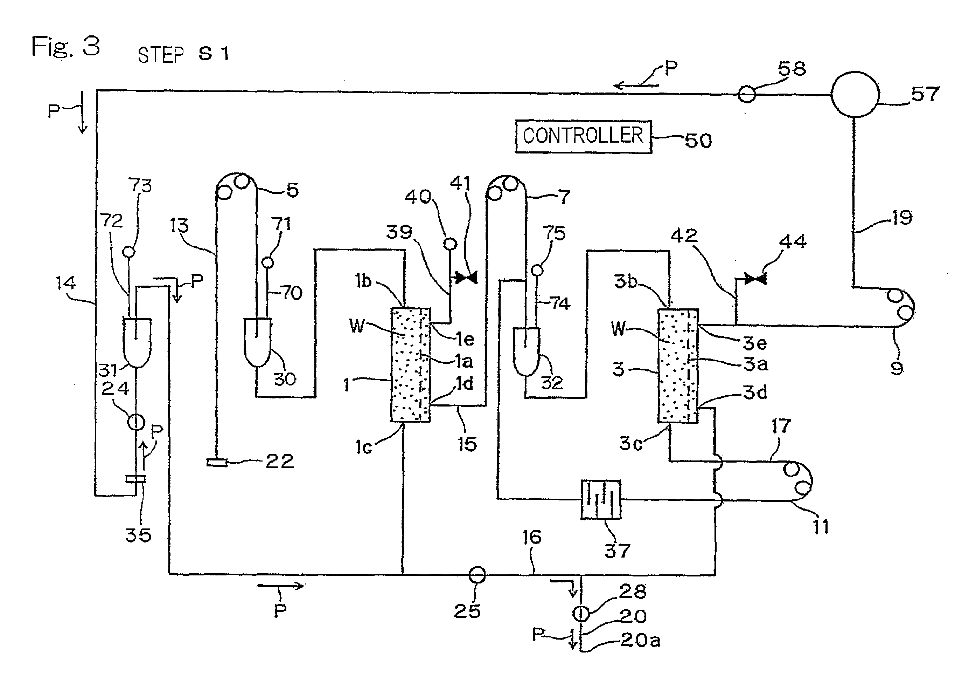

In the next place, the priming procedure for the heating type double filtration blood purification apparatus shown in Fig. 2 will be described with reference to steps S1 to S9 shown respectively in Figs. 3 to 11. Prior to the start of the priming procedure, all of the blood pump 5, the plasma introducing pump 7, the plasma discharge pump 11 and the cleansing liquid pump 9, all shown in Fig. 2, have to be checked to see if they are all halted and, at the same time, all of the blood return valve 24, the plasma return valve 25, the cleansing liquid discharge valve 28, the first air valve 41 and the second air valve 44 have to be checked to see if they are all closed. In the condition in which all of those pumps are halted and all of those valves are closed, liquid W filled within the wet type blood component separator 1, liquid W filled within the wet type plasma component separator 3 and the cleansing liquid P accommodated within the cleansing liquid supply source 57 never move anywhere within the circuit of the various passages.

At step S1 shown in Fig. 3, when the controller 50 is activated in response to a priming start signal fed from the outside to open the blood return valve 24, the plasma return valve 25 and the cleansing liquid discharge valve 28, the cleansing liquid P is discharged from the cleansing liquid supply source 57 to the outside of the apparatus by way of the cleansing liquid discharge port 20a after having sequentially flowed through the bubble sensor 58, the blood return valve 24, the return blood drip chamber 31, the plasma return valve 25 and the cleansing liquid discharge valve 28 by the effect of a pressure head resulting from the difference in level. Accordingly, air admixed in the blood return passage 14 and a portion of the plasma return passage 16 adjacent the blood delivery element 35 are purged to the outside of the apparatus and, at the same time, the blood return passage 14 and that portion of the plasma return passage 16 adjacent the blood delivery element 35 are cleansed.

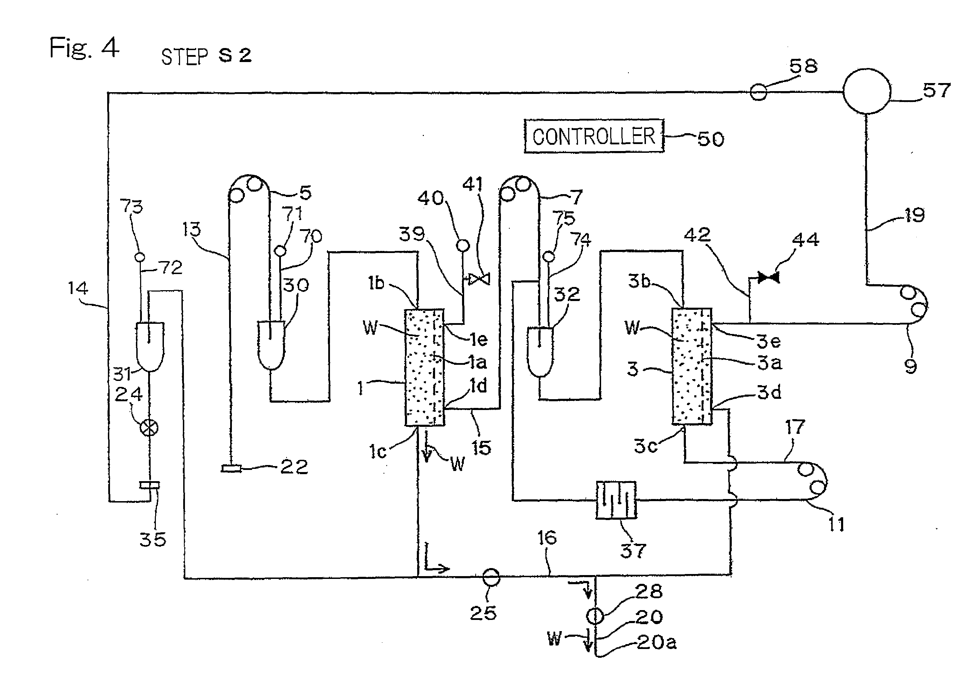

Then, at step S2 shown in Fig. 4, when the blood return valve 24 and the first air valve 41 in the first air line 39 are closed and opened, respectively, by the controller 50, air flows into the blood component separator 1 through the first air line 39 by way of the first air valve 41 and, by the effect of a pressure of the air so flowing into the blood component separator 1, the liquid W filled within the blood component separator 1 is urged to flow into the separation membrane 1a and is subsequently discharged from the cleansing liquid discharge port 20a to the outside of the apparatus after having flowed from the blood outlet 1c of the blood component separator 1, then through the plasma return valve 25 and finally through the cleansing liquid discharge valve 28 by the effect of a pressure head resulting from the difference in level. Although at this time air flows into the blood component separator 1, it will not pass across the separating membrane 1a, which is in a wet condition within the blood component separator 1, and, therefore, such air does not flow into the inside of the separator 1, that is, into a left side of the separating membrane 1a shown in Fig. 4.

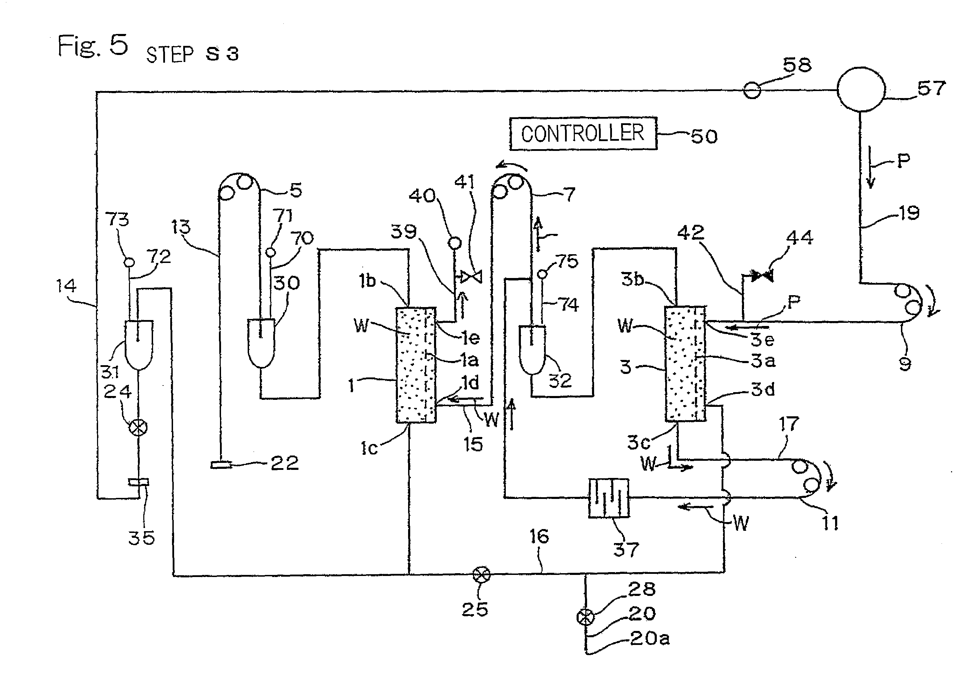

Thereafter, at step S3 shown in Fig. 5, when by means of the controller 50 the plasma valve 25 and the cleansing liquid discharge valve 28 are closed, the cleansing liquid pump 9 and the plasma discharge pump 11 are driven in a normal direction and the plasma introducing pump 7 is driven in a reverse direction, the cleansing liquid P from the cleansing liquid supply source 57 is, after the pressure thereof has been increased by the cleansing liquid pump 9, introduced into the plasma component separator 3 through the cleansing liquid inlet 3e of the plasma component separator 3. Accordingly, the liquid W filled within the plasma component separator 3 is urged by the cleansing liquid P, being then introduced into the plasma component separator 3, to flow from the first plasma component outlet 3c of the plasma component separator 3 into the high molecular plasma passage 17. The filling liquid W, which has been filled within and subsequently urged to discharge from the plasma component separator 3 in the manner described above, subsequently flows past the heater 37 into the plasma introducing passage 15 at a location upstream of the plasma drip chamber 32 in the plasma introducing passage 15 after the pressure thereof has been increased by the plasma discharge pump 11. The filling liquid W so flowing into the plasma introducing passage 15 is, after the pressure thereof has been increased by the plasma introducing pump 7, flows from the plasma outlet 1d into the blood component separator 1 and then flow upwardly through the blood component separator 1 from below.

At this time, although the cleansing liquid introducing passage 19 ad initium contains air, this air is transported to the plasma component separator 3 by way of the cleansing liquid inlet 3e of the plasma component separator 3 by the cleansing liquid P fed from the cleansing liquid supply source 57. The cleansing liquid P flowing from the cleansing liquid introducing passage 19 into the plasma component separator 3 moves the filling liquid W, then present outside the separating membrane 3a (i.e., in the right side of the separating membrane 3a shown in Fig. 5) of the plasma component separator 3, towards the inside of the separating membrane 3a (i.e., the left side of the separating membrane 3a shown in Fig. 5) by the effect of a pressure flowing in. At the time the filling liquid W and the cleansing liquid P pass across the separating membrane 3a, air present inside the separating membrane 3a is purged to the high molecular plasma passage 17 through the second plasma component outlet. The air so purged into the high molecular plasma passage 17 is transported by the filling liquid W to the plasma introducing passage 15 and is subsequently discharged from the first air valve 41 to the outside of the apparatus by way of the first air line 39 after having flowed from the plasma outlet 1d into the blood component separator 1. It is to be noted that since the air transported from the cleansing liquid introducing passage 19 to the plasma component separator 3 is incapable of passing across the separating membrane 3a which is at least wetted within the plasma component separator 3, there is no possibility that air may admix into the separating membrane 3a (in the left side of the separating membrane 3a shown in Fig. 5) from the cleansing liquid introducing passage 19.

In the meantime, the delivery of the cleansing liquid pump 9, the delivery of the plasma introducing pump 7 and the delivery of the plasma discharge pump 11 are preferably set to the same flow rates. By so setting, a sufficient pressure can be applied to the cleansing liquid P to allow the cleansing liquid P to be introduced from the plasma component separator 3 into the blood component separator 1 through the high molecular plasma passage 17 and the plasma introducing passage 15. Therefore, there is no possibility that the plasma introducing pump 7 may draw the filling liquid W within the plasma component separator 3 and the cleansing liquid P into the plasma introducing passage 15 in excess of the delivery of the cleansing liquid pump 9 and a negative pressure will hardly develop within the plasma component separator 3, the high molecular plasma passage 17 and the plasma introducing passage 15, wherefore an undesirable generation of bubbles, which will occur when elution of air inside the system or intrusion of an external air through junctions of the various passages 15, 17 and 19 with the plasma component separator 3 may be prevented. As a result, it is possible to avoid an undesirable reduction in performance of the blood component separator 1 or the plasma component separator 3 which would result from the presence of the bubbles within the separating membrane 3a of the plasma component separator 3 or within the separating membrane 1a of the blood component separator 1 after the termination of the priming.

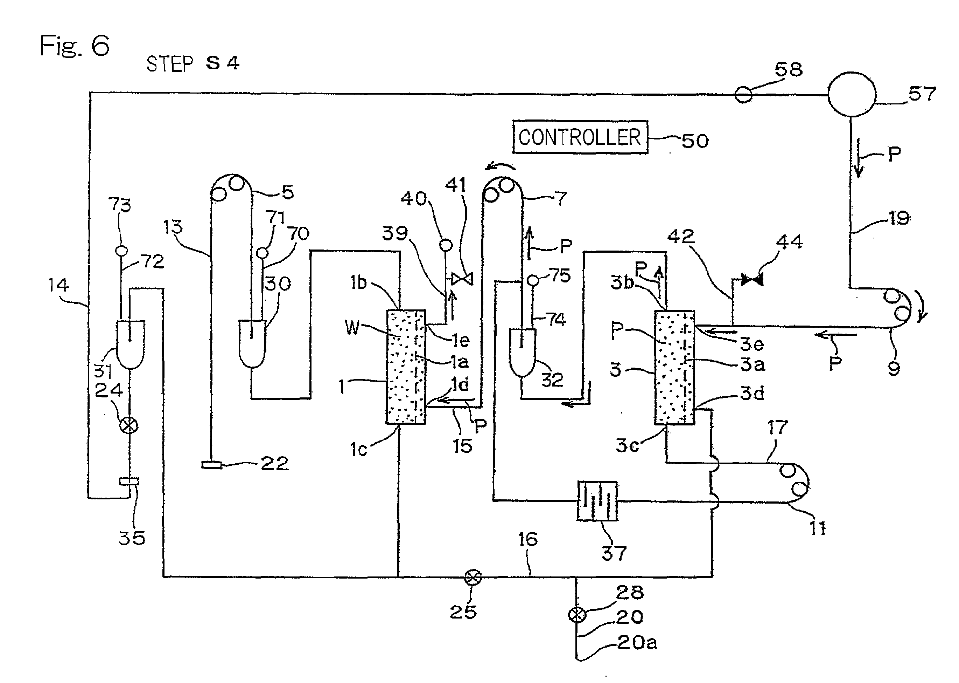

At step S4 shown in Fig. 6, the controller 50 causes the plasma discharge pump 11 to halt in a condition in which the cleansing liquid pump 9 is driven in the normal direction and the plasma introducing pump 7 is driven in the reverse direction. The cleansing liquid P introduced by the cleansing liquid pump 9 into the plasma component separator 3 flows from the plasma inlet 3a into the plasma introducing passage 15 and is, after the pressure thereof has been increased by the plasma introducing pump 7 through the plasma drip chamber 32, supplied from the plasma outlet 1d into the blood component separator 1. At this time, the respective deliveries of the cleansing liquid pump 9 and the plasma introducing pump 7 are set to the same values.

As hereinabove described, in the condition in which the rising liquid pump 9 and the plasma discharge pump 11 are driven in the normal direction and the plasma introducing pump 7 is driven in the reverse direction, a predetermined quantity of the rising liquid P is introduced into the plasma introducing passage 15, then into the plasma component separator 1 through the plasma outlet 1d and finally filled from lower side into the blood component separator 1 so as to flow upwardly therethrough. At this time, the air purged from the separating membrane 3a of the plasma component separator 3 into the plasma introducing passage 15 does, after having entered into the blood component separator 1 through the plasma outlet 1d together with the cleansing liquid P, flow upwardly through the blood component separator 1 from lower side and is subsequently discharged to the outside of the apparatus past the first air valve 41 in the first air line 39 which has been opened at step S2 shown in Fig. 4.

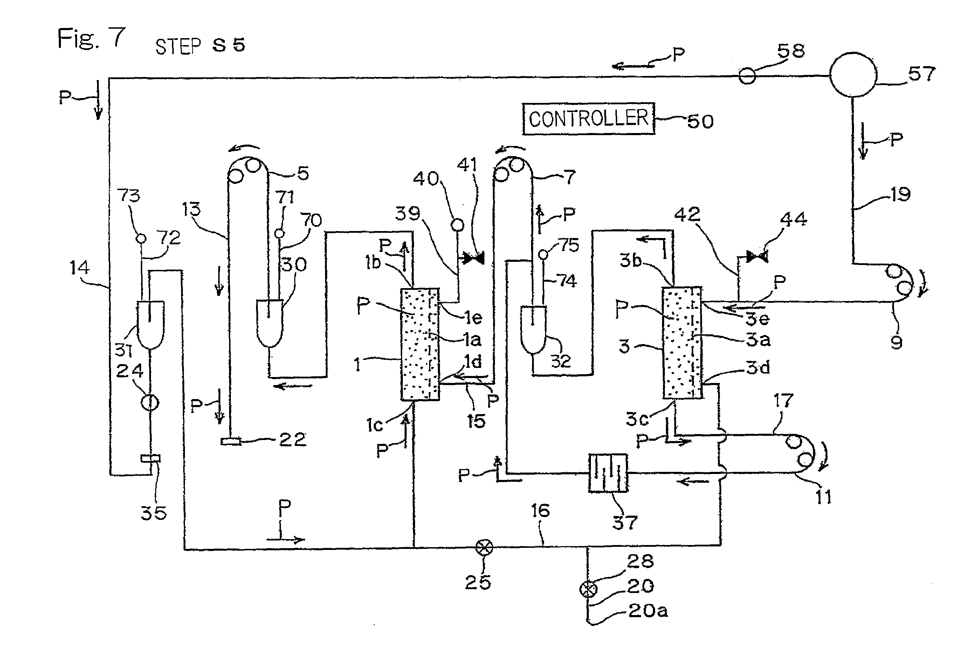

At subsequent step S5 shown in Fig. 7, when the controller 50 causes the plasma discharge pump 11 to be driven in the normal direction, the first air valve 41 to be closed, the blood return valve 24 to be opened and the blood pump 5 to be driven in the reverse direction, in a condition in which the cleansing liquid pump 9 is driven in the normal direction and the plasma introducing pump 7 is driven in the reverse direction, the cleansing liquid P flows, by the effect of a pressure head resulting from the difference in level, from the cleansing liquid supply source 57 into the separating membrane 1a of the blood component separator 1 through the return blood drip chamber 31 in the blood return passage 14 by way of the blood outlet 1c and then flows, together with the cleansing liquid P flowing from the plasma outlet 1d into the separating membrane 1a of the blood component separator 1 through the plasma introducing passage 15, from the blood inlet 1b of the blood component separator 1 into the blood introducing passage 13 after the pressure thereof has been increased by the blood pump 5. Accordingly, the air present inside the separating membrane 1a can be purged to the blood introducing passage 13 through the blood inlet 1b. Thereafter, the cleansing liquid P is discharged to the outside of the apparatus through the blood drip chamber 30, the blood pump 5 and the blood introducing element 22. Also, the blood introducing element 22 is preferably disposed at a position lower in level than those of any other passages as is the case with the branch passage 20 so that it can assume a position level with the cleansing liquid discharge port 20a.

At step S5, it is preferred that the delivery of the cleansing liquid pump 9 is set to be equal to the delivery of the plasma introducing pump 7, the delivery of the plasma discharge pump 11 is set to be smaller than the delivery of the cleansing liquid pump 9, and the delivery of the blood pump 5 is set to be larger than the delivery of the plasma introducing pump 7. By so setting, no negative pressure is developed inside any one of the plasma component separator 3, the plasma introducing circuit 15 the blood return passage 14 and the blood introducing passage 13 and, therefore, generation of the bubbles resulting from elution of air from the inside of the system and ingress of an external air from the outside can be prevented advantageously.

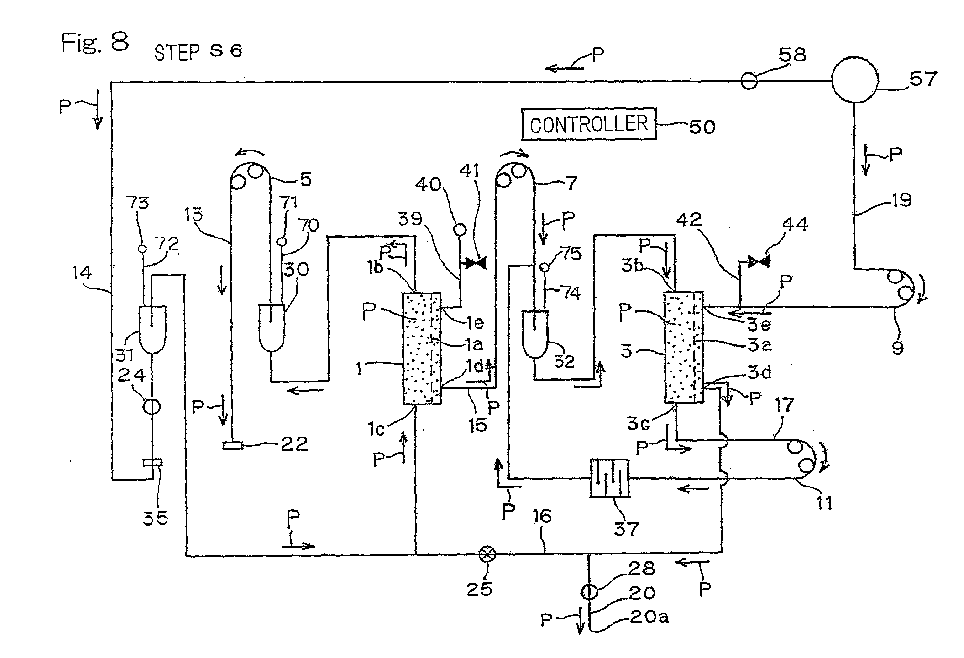

Thereafter, at step S6 shown in Fig. 8, when the controller 50 causes the cleansing liquid discharge valve 28 to open and the plasma introducing pump 7 to be driven in the normal direction, a portion of the cleansing liquid P flowing into the blood component separator 1 flows into the plasma introducing passage 15 through the plasma outlet 1d and is then introduced into the plasma component separator 3 through the plasma introducing pump 7 and the plasma drip chamber 32. When at this time, the plasma introducing pump 7 is driven in the normal direction at the same delivery as that of the cleansing liquid pump 9 and the plasma discharge pump 11 is driven in the normal direction at a delivery lower than that of the plasma introducing pump 7 and the cleansing liquid pump 9, the cleansing liquid P can be sufficiently passed upwardly from below to the separating membrane 3a of the plasma component separator 3. A large amount of the cleansing liquid P having flowed through the separating membrane 3a flows into the plasma return passage 16 and the branch passage 20 through the second plasma component outlet 3d and is then discharged to the outside of the apparatus through the cleansing liquid discharge valve 28. On the other hand, the remaining amount of the cleansing liquid P flows into the high molecular plasma passage 17 through the first plasma component outlet 3c and is, after the pressure thereof has been increased by the plasma discharge pump 11 through the heater 37, merged with the cleansing liquid P then flowing in the plasma introducing passage 15.

After a predetermined quantity of cleansing liquid P has been flowed at the above described preset deliveries (step S6), the respective deliveries of the blood pump 5, the plasma introducing pump 7, the plasma discharge pump 11 and the cleansing liquid pump 9 are so set properly that no negative pressure may be developed within the blood component separator 1, the plasma component separator 3 and the various passages. By so doing, the blood component separator 1, the plasma component separator 3, the blood introducing passage 13, the plasma introducing passage 15, the plasma return passage 15 and the high molecular plasma passage 17 are cleansed sufficiently. It is to be noted that since the plasma return valve 25 is closed, the cleansing liquid P, which has flown in the plasma return passage 16 and has therefore been contaminated, flows into the blood return passage 14 and the blood return passage 14 will not be contaminated.

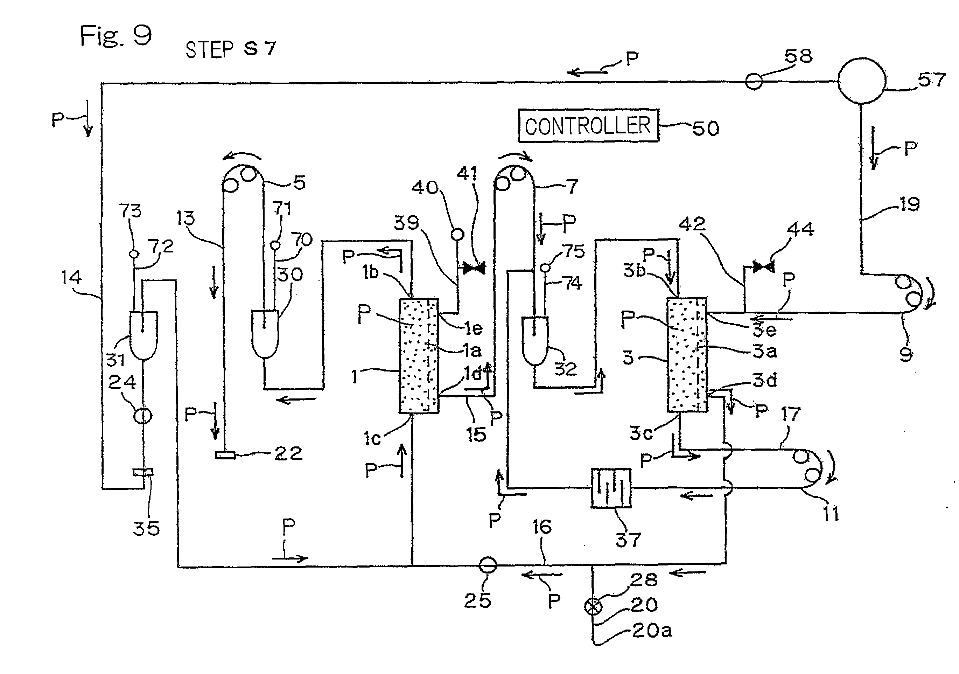

At step S7 shown in Fig. 9, the controller 50 causes the cleansing liquid discharge valve 28 to be closed and the plasma return valve 25 to be opened. At this time, the delivery of the blood pump 5 then driven in the reverse direction is set to a value higher than the delivery of the cleansing liquid pump 9 and, at the same time, the respective deliveries of the plasma introducing pump 7, and the plasma discharge pump 11, both then driven in the normal direction, are so properly set that no negative pressure will not develop within the plasma introducing passage 15 and the high molecular plasma passage 17. Accordingly, the entire circuit of the system excluding the blood component separator 1, the plasma component separator 3 and the branch passage 20 can be cleansed and refilled with the cleansing liquid P.

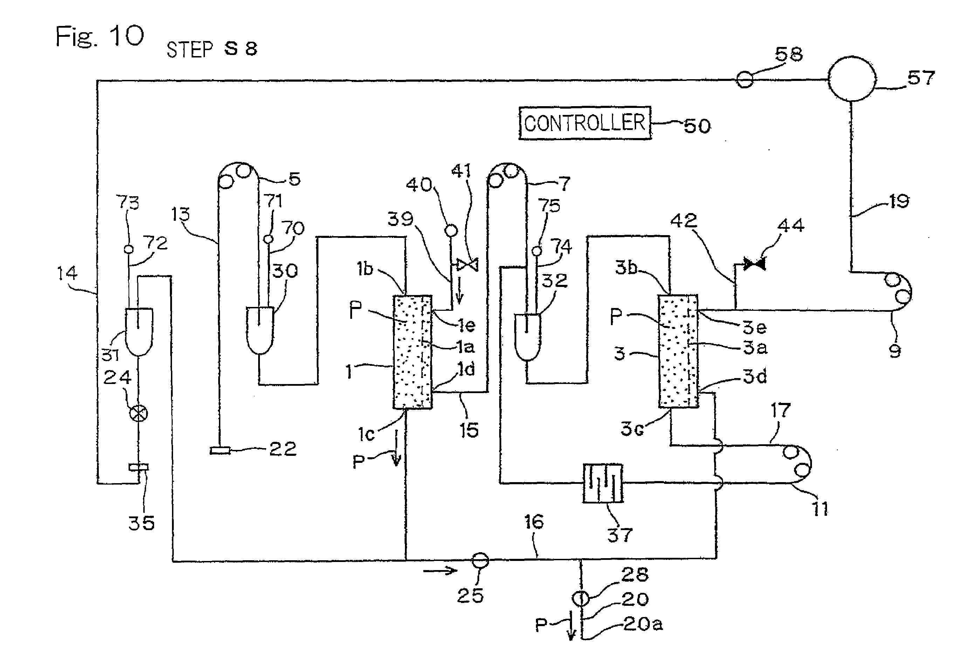

Thereafter, at step S8 shown in Fig. 10, when the controller 50 causes all of the pumps to halt, the blood return valve 24 and the second air valve 44 to be closed and the plasma return valve 25, the cleansing liquid discharge valve 28 and the first air valve 41 to be opened, the cleansing liquid P filled outside the separation membrane 1a (the right side of the separation membrane 1a shown in Fig. 10) of the blood component separator 1 is discharged to the outside of the apparatus through the cleansing liquid discharge valve 28 in the branch passage 20 by the effect of the gravitational force.

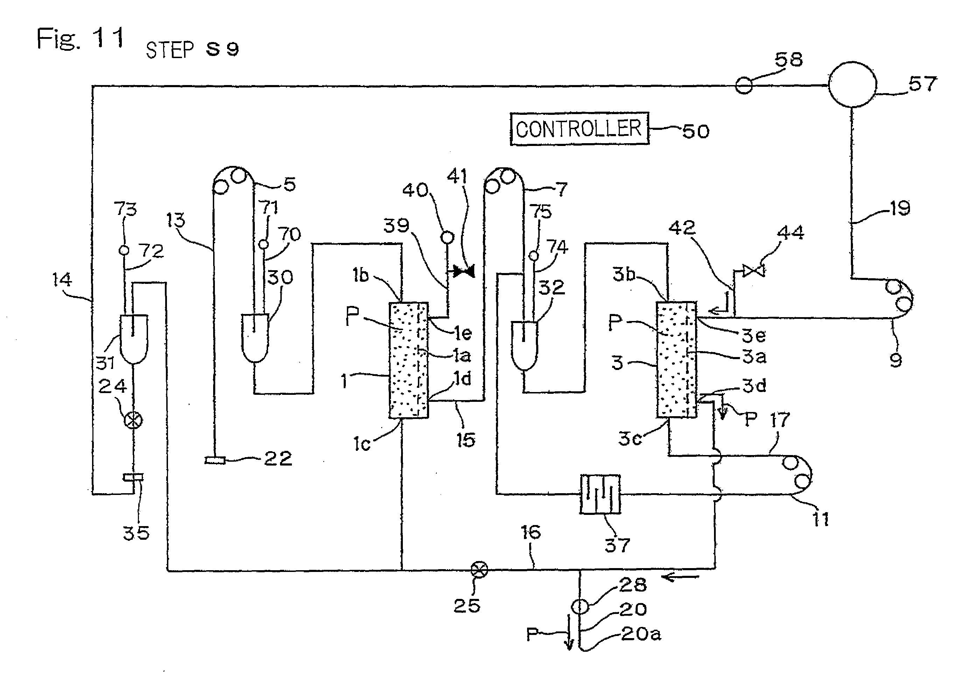

Finally, at step S9 shown in Fig. 11, when the controller 50 causes the plasma return valve 25 and the first air valve 41 to be closed and the second air valve 44 to be opened, the cleansing liquid P filled outside the separating membrane 3a (the right side of the separating membrane 3 a shown in Fig. 11) of the plasma component separator 3 is discharged to the outside of the apparatus through the cleansing liquid discharge valve 28 in the branch passage 20 by the effect of the gravitational force. In this way, the priming procedure completes.

The cleansing liquid introducing passage 19 is capable of being selectively connected with the cleansing liquid supply source 57 shown in Fig. 2 and a liquid replacement supply source 60 reserving a quantity of liquid replacement 60a to be supplemented to the patient. Accordingly, the cleansing liquid introducing passage 19 is used as a circuit for introducing the cleansing liquid P from the cleansing liquid supply source 57 into the plasma component separator 3 during the priming taking place, but during the clinical treatment the liquid replacement 60a is introduced from the liquid replacement supply source 60 into the plasma component separator 3 to that the cleansing liquid introducing passage 19 can be used as a liquid replacement introducing circuit by which the liquid replacement can be added to the low molecular weight subcomponent. Accordingly, there is no need to employ any dedicated circuit for adding the liquid replacement 60a to the low molecular weight subcomponent and, therefore, the double filtration blood purification apparatus can be advantageously simplified in structure.

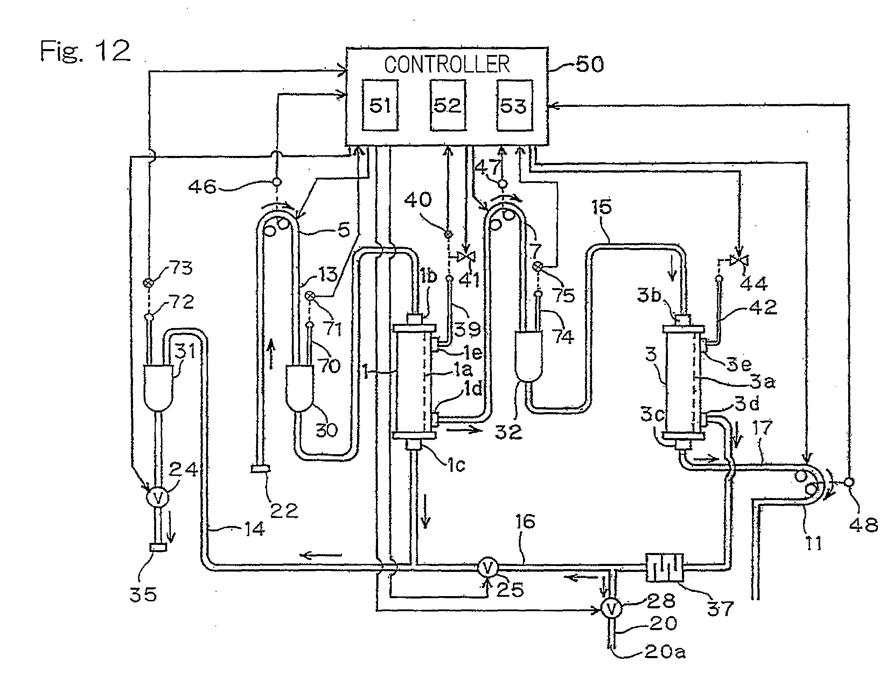

Fig. 12 illustrates a schematic diagram showing the double filtration blood purification apparatus according to a different preferred embodiment of the present invention. This double filtration blood purification apparatus is of a type, in which a circuit for recirculating the high molecular plasma, which is employed in the heating type double filtration blood purification apparatus shown in and described with particular reference to Fig. 1, is dispensed with and, in such case, the high molecular plasma passage 17 is provided with a plasma discharge pump 11. Also, the heater 37, which has been shown and employed in the high molecular plasma passage 17 in the heating type double filtration blood purification apparatus shown in and described with reference to Fig. 1, is provided upstream of the plasma return valve 25 in the plasma return passage 16. Other structural features than those described above are identical with those in the heating type double filtration blood purification apparatus shown in and described with particular reference to Fig. 1 and, therefore, the details thereof are not reiterated for the sake of brevity.

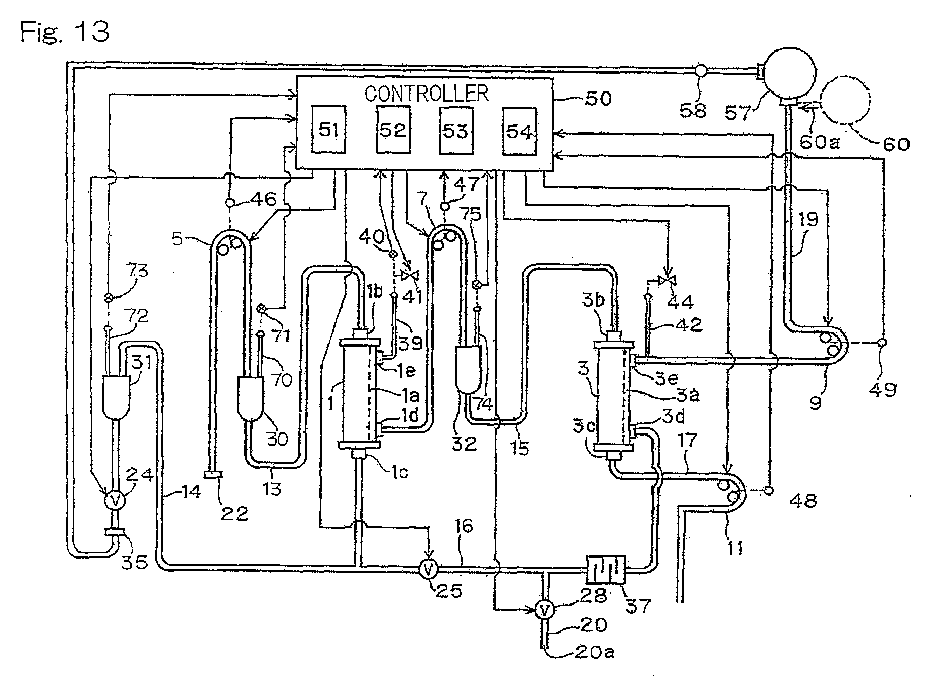

Fig. 13 illustrates a schematic diagram of the double filtration blood purification apparatus of Fig. 12, showing the manner in which the apparatus is primed and, as is the manner in which the heating type double filtration blood purification apparatus shown in and described with particular reference to Fig. 2 is primed, the plasma component separator 3 is fluidly connected with the cleansing liquid introducing passage 19 so that the priming procedure can be performed. Also, this cleansing liquid introducing passage 19 is similarly provided with the cleansing liquid pump 9.

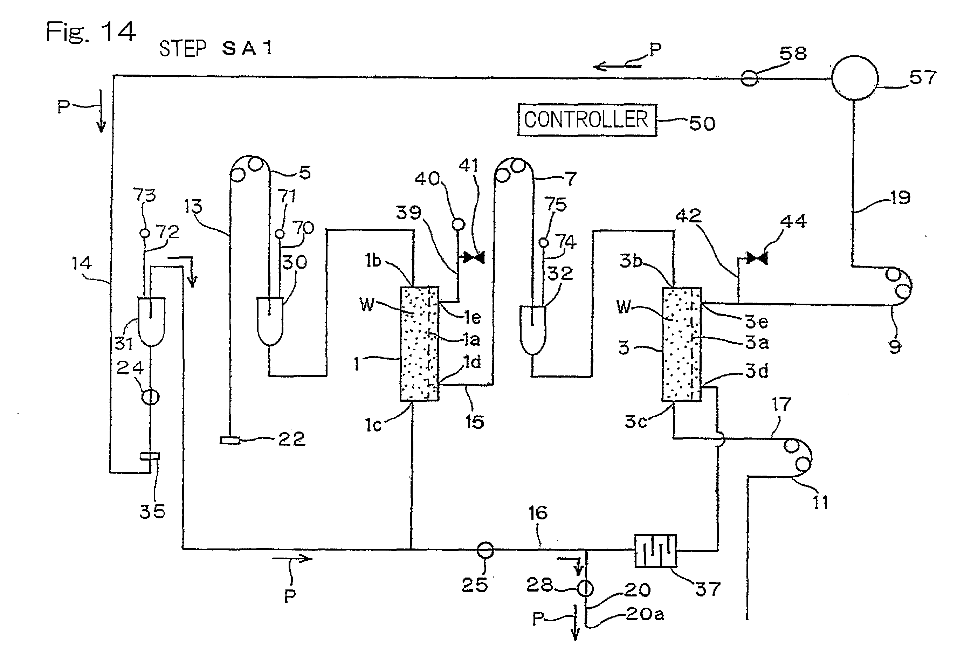

In the next place, the priming procedure performed by the double filtration blood purification apparatus of the construction shown in Fig. 13 will be described in detail with particular reference to Figs. 14 to 21 showing respective steps SA1 to SA8. Prior to the priming being initiated, as is the case with the first embodiment hereinbefore described, check is made to see if all of the pumps are halted and all of the valves are closed.

At step SA1 shown in Fig. 14, the controller 50 is activated in response to receipt of a priming start signal from the outside, and when by this controller 50 so activated, the blood return valve 24, the plasma return valve 25 and the cleansing liquid discharge valve 28 are opened, the cleansing liquid P from the cleansing liquid supply source 57 is, by the effect of a pressure head resulting from the difference in level, discharged to the outside of the apparatus from the cleansing liquid discharge port 20a after having flowed through the bubble sensor 58, the blood return valve 24, the return blood drip chamber 31 and the plasma return valve 25 and finally through the cleansing liquid discharge valve 28. Accordingly, air mixing in respective portions of the blood return passage 14 and the plasma return passage 16 adjacent the blood delivery element 35 are discharged to the outside of the apparatus and, at the same time, those portions of the blood return passage 14 and the plasma return passage 16 adjacent the blood delivery element 35 are cleansed.

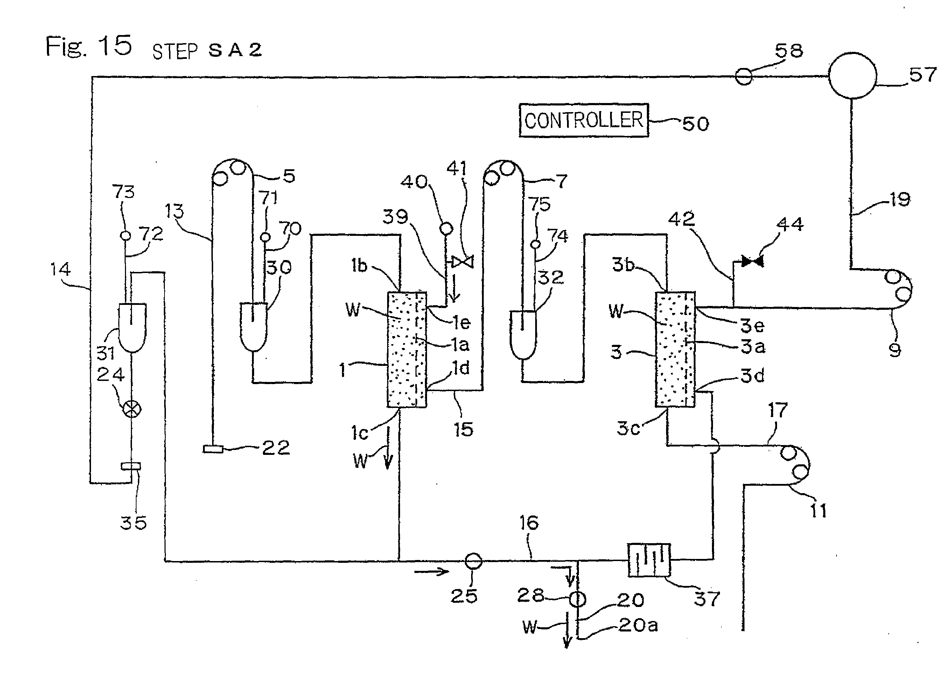

Then, at step SA2 shown in Fig. 15, when the controller 50 causes the blood valve 24 to be closed and the first air valve 41 in the first air line 39 to be opened, air flows from the first air line 39 into the blood component separator 1 by way of the first air valve 41 and, by the effect of a pressure of the air so flowing in, liquid W filled within the blood component separator 1 flows into the inside of the separating membrane 1a and is then discharged to the outside of the apparatus from the blood outlet 1c of the blood component separator 1 by way of the cleansing liquid discharge port 20a through the plasma return valve 25 and then through the cleansing liquid discharge valve 28 by the effect of the difference in level. Although at this time, air flows into the blood component separator 1, since the air cannot pass through the separating membrane 1a of the blood component separator 1, which is then held in a wetted condition, no air admixes into the inside of the separating membrane 1a, that is, into the left side of the separating membrane shown in Fig. 15.

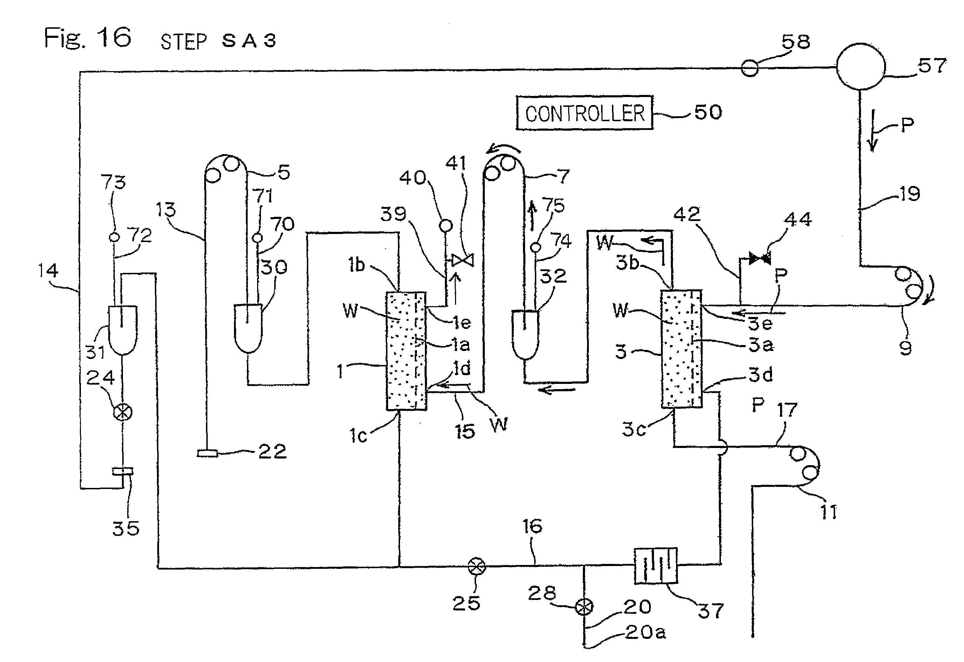

Thereafter, at step SA3 shown in Fig. 16, when the controller 50 causes the plasma return valve 25 and the cleansing liquid discharge valve 28 to be closed, the cleansing liquid pump 9 to be driven in the normal direction and the plasma introducing pump 7 to be driven in the reverse direction, the cleansing liquid P from the cleansing liquid supply source 57 is, after the pressure thereof has been increased by the cleansing liquid pump 9, introduced into the plasma component separator 3 through the cleansing liquid inlet 3e of the plasma component separator 3 and, then, flows into the plasma introducing passage 15 through the plasma inlet 3b of the plasma component separator 3. The cleansing liquid P flowing into the plasma introducing passage 15 in the manner described above flows into the blood component separator 1 from the plasma outlet 1d, after the pressure thereof has been increased by the plasma introducing pump 7, and then flows upwardly through the blood component separator 1 from below. Since at this time the cleansing liquid P is, after having been pressurized sufficiently by the cleansing liquid pump 9, introduced into the plasma component separator 3, the liquid W filled within the plasma component separator 3 is moved towards the inside of the separating membrane 3a (into the left side of the separating membrane 3a shown in Fig. 16) to thereby urge it into the plasma introducing passage 15 through the plasma inlet 3b and, at the same time, during the passage of the cleansing liquid P itself through the separating membrane 3a, air present inside the separating membrane 3a is urged into the plasma introducing passage 15 through the plasma inlet 3b. The air so urged into the plasma introducing passage 15 moves upwardly through the blood component separator 1 from below together with the filling liquid W and the cleansing liquid P and is subsequently discharged to the outside of the apparatus through the first air valve 41 in the first air line 39.

Although at this time air is at first present within the cleansing liquid introducing passage 19, this air is transported by the cleansing liquid P, fed from the cleansing liquid supply source 57, into the plasma component separator 3 through the cleansing liquid inlet 3e of the plasma component separator 3. The cleansing liquid P flowing from the cleansing liquid introducing passage 19 into the plasma component separator 3 in this manner causes the filling liquid W present outside the separating membrane 3a (the right side of the separating membrane 3a shown in Fig. 16) of the plasma component separator 3 to move into the inside of the separating membrane 3a (into the left side of the separating membrane 3a shown in Fig. 16) by the effect of the pressure so flowing thereinto. The filling liquid W and the cleansing liquid P urges the air present inside the separating membrane 3a into the plasma introducing passage 15 through the plasma inlet 3b as they flow through the separating membrane 3a. The air so urged into the plasma introducing passage 15 in the manner described above is transported by the filling liquid W, then flowing from the plasma inlet 3b into the plasma introducing passage 15, so as to flow from the plasma outlet 1d into the blood component separator 1 and is then discharged to the outside of the apparatus through the first air valve 41 by way of the first air line 39. It is to be noted that since the air transported from the cleansing liquid introducing passage 19 into the plasma component separator 3 is incapable of passing through the separating membrane 3a, which is at least in a wetted condition within the plasma component separator 3, air flow into the separating membrane 3a (in the left side of the separating membrane 3a shown in Fig. 5) from the cleansing liquid introducing passage 19 may be prevented.

In the meantime, the delivery of the cleansing liquid pump 9 is preferably set to the same flow rate as that of the plasma introducing pump 7. By so setting, a sufficient pressure can be applied to the cleansing liquid P to allow the cleansing liquid P to be introduced from the plasma component separator 3 into the blood component separator 1 through the plasma introducing passage 15 and, therefore, the plasma introducing pump 7 does not draw the filling liquid W and the cleansing liquid P within the plasma component separator 3 into the plasma introducing passage 15 in excess of the delivery of the cleansing liquid pump 9 and a negative pressure will hardly develop within the plasma component separator 3 and the plasma introducing passage 15, whereby an undesirable generation of bubbles, which will occur when elution of air inside the system or intrusion of an external air through junctions of the passages 15 and 19 with the plasma component separator 3 may be prevented. As a result, it is possible to avoid an undesirable reduction in performance of the blood component separator 1 or the plasma component separator 3 which would result from the presence of the bubbles within the separating membrane 3a of the plasma component separator 3 or within the separating membrane 1a of the blood component separator 1.

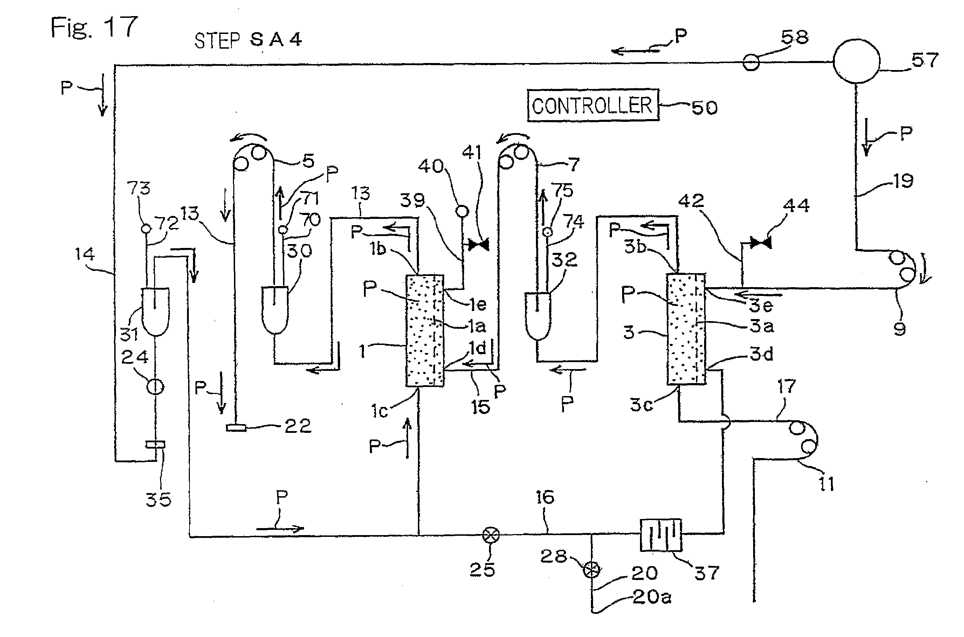

Furthermore, at step SA4 shown in Fig. 17, the controller 50 causes the first air valve 41 to be closed, the blood return valve 24 to be opened and the blood introducing pump 5 to be driven in the reverse direction, while in a condition in which the cleansing liquid pump 9 to be driven in the normal direction and the plasma introducing pump 7 is driven in the reverse direction. The cleansing liquid P flowing in the blood return passage 14 according to the difference in level is introduced into the blood component separator 1 as a result of increase in pressure brought about by the blood pump 5 then driven in the reverse direction, and is subsequently merged with the cleansing liquid P flowing in the plasma introducing passage 15 before it enters the blood introducing passage 13. The cleansing liquid P entering the blood introducing passage 13 in the manner described above is discharged to the outside of the apparatus through the blood drip chamber 30 and the blood pump 5. At this time, by setting the delivery of the cleansing liquid pump 9 to a value equal to the delivery of the plasma introducing pump 7 and, also, the delivery of the blood introducing pump 5 to a value larger than the delivery of the plasma introducing pump 7, it is possible to avoid an undesirable development of a negative pressure within the blood component separator 1, the plasma component separator 3, the blood introducing passage 13, the blood return passage 14 and the plasma introducing passage 15. Also, the air remaining within the separating membrane 3a of the plasma component separator 3 can be purged to the plasma introducing passage 15.

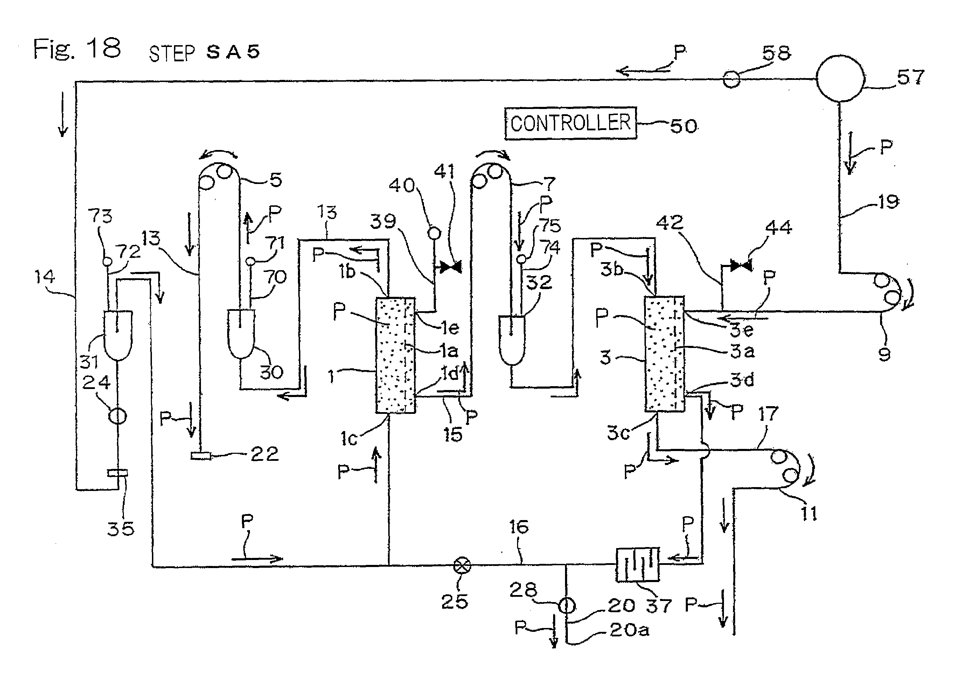

Subsequently, at step SA5 shown in Fig. 18, when the controller 50 causes the plasma introducing pump 7 and the plasma discharge pump 11 to be driven in the normal direction in a condition in which the plasma introducing pump 5 is driven in the reverse direction and the cleansing liquid pump 9 is driven in the normal direction, respectively, the cleansing liquid P, which has flowed into the blood component separator 1 through the blood return passage 14 and has subsequently entered the plasma introducing passage 15 after the pressure thereof had been increased by the plasma introducing pump 7, is discharged by the plasma discharge pump 11 to the outside of the apparatus from the plasma component separator 3 and, at the same time, a portion of such cleansing liquid P introduced into the plasma component separator 3 flows in the blood return passage 16 through the second plasma component outlet 3d and is then discharged to the outside of the apparatus past the cleansing liquid discharge valve 28, wherefore the plasma component separator 3 is cleansed. At this time, the delivery of the plasma discharge pump 11 is set to a value smaller than total sum of the delivery of the plasma introducing pump 7 and the delivery of the cleansing liquid pump 9, preferably to a value equal to about 1/2 of such sum of the deliveries. By so doing, no negative pressure will develop within the blood component separator 1, the plasma component separator and the various passages and, therefore, generation of the bubbles can be avoided.

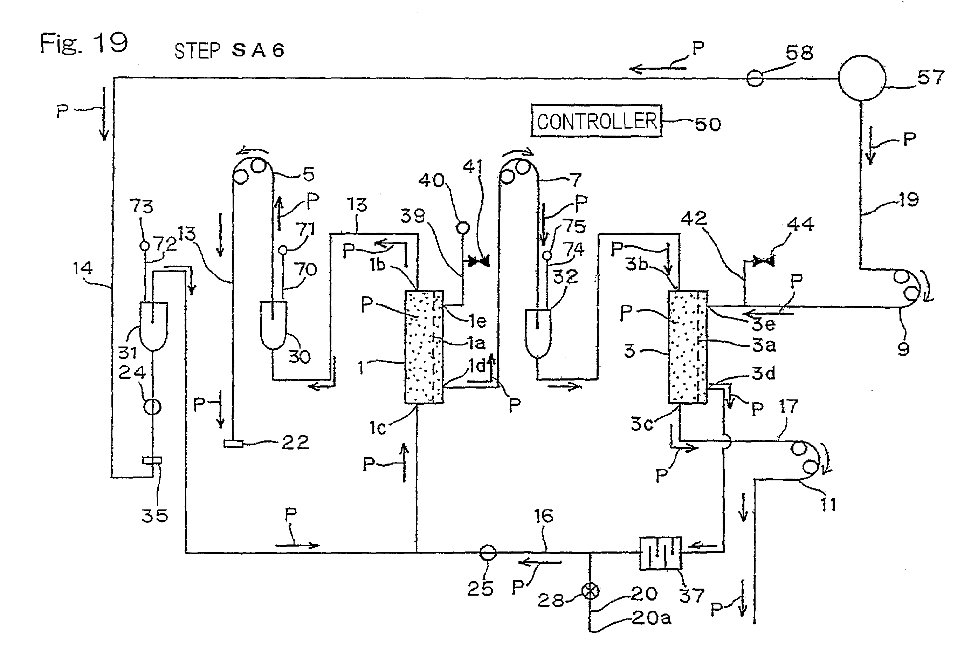

Then, at step SA6 shown in Fig. 19, when the controller 50 causes the cleansing liquid discharge valve 28 to be closed and the plasma return valve 25 to be opened, the cleansing liquid P within the plasma component separator 3 flows from the second plasma component outlet 3d into the plasma return passage 16 and, therefore, the plasma return passage 16 is cleansed. When during this condition the priming procedure is continued, the blood component separator 1, the plasma component separator 3 and the various passages can be sufficiently cleansed.

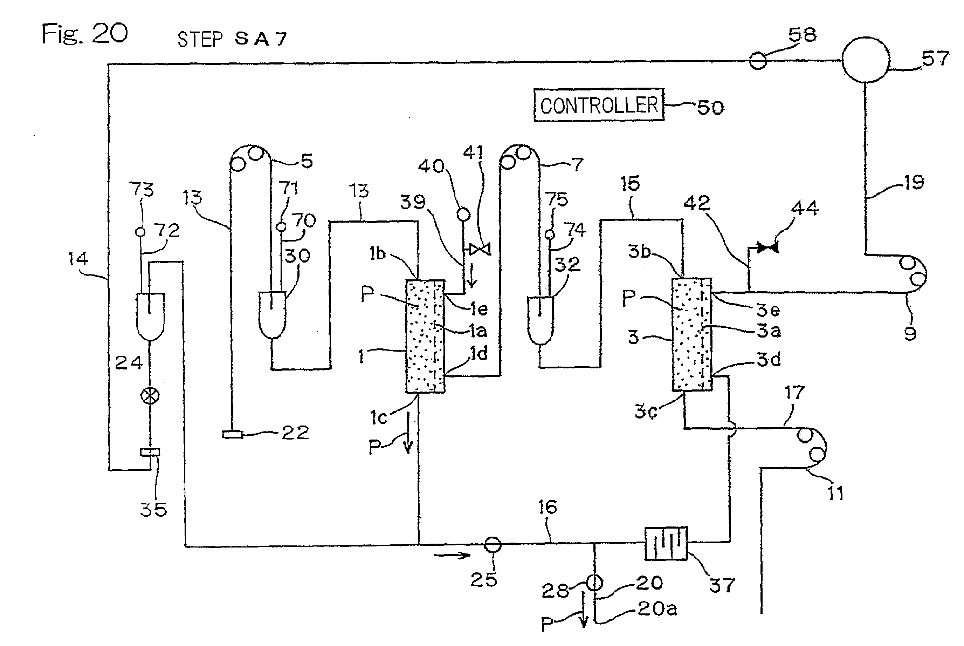

Thereafter, at step SA7 shown in Fig. 20, when in the condition, in which the plasma return valve 25 is opened, the controller 50 causes all of the pumps to be halted, the blood return valve 24 and the second air valve 44 to be closed and the cleansing liquid discharge valve 28 and the first air valve 41 to be opened, the cleansing liquid P within the blood component separator 1 is discharged to the outside of the apparatus through the cleansing liquid discharge valve 28 in the branch passage 20.

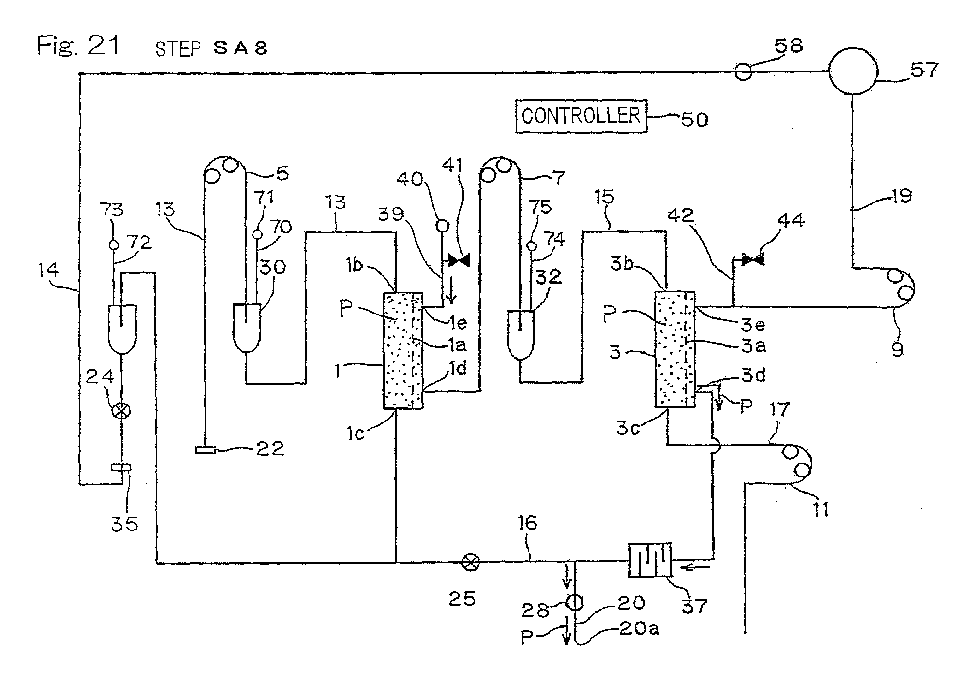

Finally, at step SA8 shown in Fig. 21, when the controller 50 causes the plasma return valve 25 and the first air valve 41 to be closed and the cleansing liquid discharge valve 28 and the second air valve 44 to be opened, the cleansing liquid P within the plasma component separator 3 is discharged to the outside of the apparatus through the cleansing liquid discharge valve 28 in the branch passage 20. Thereafter, when all of the valves are closed, the priming procedure completes.

Although the present invention has been fully described in connection with the preferred embodiments thereof with reference to the accompanying drawings which are used only for the purpose of illustration, those skilled in the art will readily conceive numerous changes and modifications within the framework of obviousness upon the reading of the specification herein presented of the present invention. Accordingly, such changes and modifications are, unless they depart from the scope of the present invention as delivered from the claims annexed hereto, to be construed as included therein.

## Claims

1. A double filtration blood purification apparatus, which comprises:
a blood component separator 1 for separating a blood into a blood cell component and a plasma component;
a plasma component separator 3 for separating the plasma component into a high molecular weight subcomponent and a low molecular weight subcomponent;
a plasma introducing passage 15 fluidly connected with the blood component separator 1 and the plasma component separator 3 for introducing the plasma component into the plasma component separator 3;
a high molecular plasma passage 17 for discharging the separated high molecular weight subcomponent from the plasma component separator 3;
a cleansing liquid introducing passage 19 fluidly connected with the plasma component separator 3 for introducing a cleansing liquid into the plasma component separator 3;
a first pump 7 disposed in the plasma introducing passage 15;
a second pump 11 disposed in the high molecular plasma passage 17;
a third pump 9 disposed in the cleansing liquid introducing passage 19; and
a controller 50 for controlling the first pump 7, the second pump 11 and the third pump 9;
wherein the controller 50 is operable during a priming to cause the third pump 9 to be driven in a normal direction and the first pump 7 to be driven in a reverse direction to thereby allow the cleansing liquid to flow from the cleansing introducing passage 19 into the plasma component separator 3 and also to flow from the plasma component separator 3 to the blood component separator 1 through the plasma introducing passage 15.

2. The double filtration blood purification apparatus as claimed in Claim 1, further comprising an air valve 41 for discharging air from the blood component separator 1 to an outside of the apparatus and wherein the controller 50 is operable during the priming to cause the air valve 41 to be opened.

3. The double filtration blood purification apparatus as claimed in Claim 1, wherein the third pump 9 has a delivery larger than that of the first pump 7.

4. The double filtration blood purification apparatus as claimed in Claim 1, wherein the cleansing liquid introducing passage 19 is set to be selectively connected during a clinical treatment with a liquid replacement supply source for supplying a liquid replacement to the low molecular weight subcomponent.

5. The double filtration blood purification apparatus as claimed in Claim 1, wherein the separated plasma component is introduced from upper side into the plasma component separator 3.

6. A method of priming a double filtration blood purification apparatus, which comprises:
separating a blood into a blood cell component and a plasma component by means of a blood component separator 1;
introducing the separated plasma component into a plasma component separator 3 through a plasma introducing passage 15;
separating the plasma component into a high molecular weight subcomponent and a low molecular weight subcomponent by means of a plasma component separator 3;
discharging the separated high molecular weight subcomponent from the plasma component separator 3 through a high molecular plasma passage 7;
introducing a cleansing liquid from a cleansing liquid introducing passage 19 into the plasma component separator 3;
providing a first pump 7 in the plasma introducing passage 15;
providing a second pump 11 in the high molecular plasma passage 17; and
providing a third pump 9 in the cleansing liquid introducing passage 19;
wherein during priming, the third pump 9 is driven in a normal direction and the first pump 7 is driven in a reverse direction to thereby allow the cleansing liquid to flow from the cleansing liquid introducing passage 19 into the plasma component separator 3 and, also, from the plasma component separator 3 into the blood component separator 1 through the plasma introducing passage 15.

7. The method of priming the double filtration blood purification apparatus as claimed in Claim 6, further comprising providing an air valve 41 for discharging air from the blood component separator 1 to an outside of the apparatus, the air valve 41 being opened during the priming.

8. The method of priming the double filtration blood purification apparatus as claimed in Claim 6, wherein the third pump 9 has a deliverieset to be larger than that of the first pump 7.

9. The method of priming the double filtration blood purification apparatus as claimed in Claim 6, wherein during a clinical treatment the cleansing liquid introducing passage 19 is used as a passage through which a liquid replacement is allowed to flow so that the liquid replacement is added to the low molecular weight subcomponent.

10. The method of priming the double filtration blood purification apparatus as claimed in Claim 6, wherein the separated plasma component is introduced from upper side into the plasma component separator 3.
